# EUROPEAN PATENT SPECIFICATION

(11) **EP 0 952 140 B1**
(45) Date of publication and mention of the grant of the patent: **28.04.2004**
(21) Application number: 99810199.2
(22) Date of filing: 08.03.1999
(51) Int. Cl.: C07C 49/17, C07C 45/42, A23L 1/226

(54) **2-Hydroxy-5-methyl-hexan-3-one and 3-hydroxy-5-methyl-hexan-2-one**
2-Hydroxy-5-methyl-hexan-3-on und 3-hydroxy-5-methyl-hexan-2-on
2-Hydroxy-5-méthyl-hexan-3-one et 3-hydroxy-5-méthyl-hexan-2-one

(30) Priority: 20.04.1998 EP 98810338
(43) Date of publication of application: 27.10.1999
(73) Proprietor: Givaudan SA, 1214 Vernier-Genève (CH)
(72) Inventor: Gautschi, Markus, Dr., 4314 Zeiningen (CH); Ibanez, Luis, 8600 Dübendorf (CH)
(74) Representative: McStea, John Anthony

(56) References cited:
- CHEMICAL ABSTRACTS, vol. 122, no. 23, 5 June 1995 Columbus, Ohio, US; abstract no. 289314, MOIO, L. ET AL: "3-Hydroxy-5-methyl-2-hexanone, a new compound characterized by a melted cheese flavor in dairy products" XP002103223 & ITAL. J. FOOD SCI. (1994), 6(4), 441-7 CODEN: ITFSEY;ISSN: 1120-1770,
- DATABASE WPI Section Ch, Week 8807 Derwent Publications Ltd., London, GB; Class E14, AN 88-046873 XP002103224 & JP 63 005050 A (DAICEL CHEM IND LTD) , 11 January 1988

## Description

The present invention relates to hydroxy-methyl-hexanones namely to 2-hydroxy-5-methyl-hexan-3-one and mixtures of this compound, with 3-hydroxy-5-methyl-hexan-2-one, flavoring and/or masking compositions, food or beverage flavored with the above compounds and a process for the preparation of said compounds.

The skilled person in the food and beverage industry knows of the critical role flavor plays in the appreciation of food and beverage. Many food products, such as cocoa, chocolate, coffee, caramel, nuts, malt and the like have flavor quality that is referred to in the language of this art as "roasted brown". In the following the term "roasted brown" will be used to describe flavor properties of the mentioned food products as well as of food products having similar flavor characteristics. The term flavor comprises in the following flavor, aroma and taste.

From the above group of food products, flavor is especially important for chocolate. Fine flavor, high nutritional value, pleasing appearance and good storage qualities make chocolate to a food product of exceptional value which is very popular and widely used. The flavor of chocolate depends on the quality and origin of the cocoa beans, on the processing thereof and the preparation of the chocolate. The processing steps which influence the chocolate quality, include fermentation, drying, roasting, cleaning and milling of the cocoa beans. In the preparation of the chocolate the ratio of cocoa mass, sugar and cocoa butter, etc. determines the flavor of the product. The flavor of chocolate has been thoroughly investigated and many volatiles contributing to the overall flavor have been determined (I. Flament, "Coffee, cacao and tea", in Volatile compounds in foods and beverages, H. Maarse, Ed., Marcel Dekker, Inc. New York, 1991; Grosch, Lehrbuch der Lebensmittelchemie, 4. Ed. 1997). Compounds mainly contributing to the cocoa flavor are aldehydes, especially isovaleraldehyde and phenylethanal as well as the corresponding aldol condensation product 5-methyl-2-phenyl-2-hexenal.

Many chocolate and cocoa based foods and beverages produced by the food industry lack flavor characteristics of high quality chocolate and cocoa. A great deal of effort was used to provide compounds with a natural chocolate or cocoa flavor for imparting the flavor of high quality chocolate and cocoa to products of low quality. US Patent 3,582,360 discloses unsaturated aldehydes, especially 2-phenyl-2-alkenals, for preparing flavor compositions and food products, particularly with chocolate and cocoa flavor.

Also other food products with roasted brown flavor lack this freshly processed flavor. This lack substantially reduces the overall organoleptic impression of the freshly roasted product. The missing flavor note is described as green, pungent and cocoa-like. The deficiency in roasted brown flavor is mainly due to a decrease of isovaleraldehyde. Isovaleraldehyde is relatively highly volatile, has a high reactivity towards alcohols, such as propylene glycol used as solvent in said flavorings and undergoes aldol reactions with other aldehydes, such as phenylethanal, present in the flavorings.

Object of the present invention is to provide compounds which are useful in imparting roasted brown flavor to food and beverage products.

This object is achieved with one novel compound of formula I 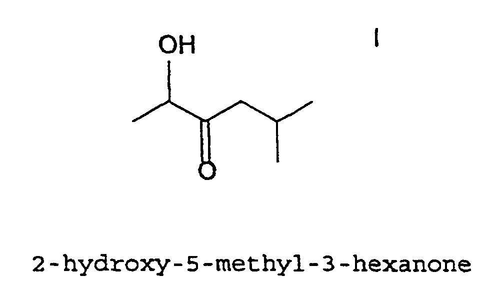 and a novel mixture of the compound of formula I with a compound of formula II 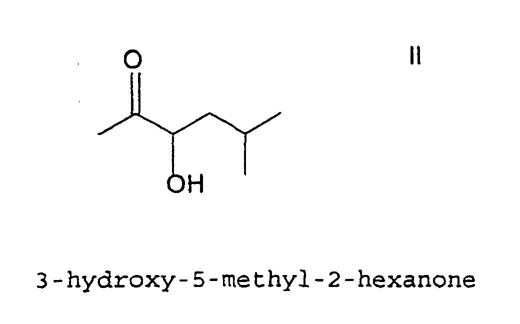 which impart similar sensory flavor impression to isovaleraldehyde to roasted brown flavorings, food products and beverages. They are especially useful to enhance the flavor of flavoring compositions, food and beverage products with roasted brown flavoring characteristics.

α-hydroxyketones are well represented in food products. One example is acetoin (3-hydroxy-2-butanone) having a buttery, creamy character which can be found in a great number of food products (Volatile compounds in food, qualitative and quantitative data, TNO Nutrition and Food Research, supplement 5, H. Maarse, C.A. Visscher, L.C. Willemsens, L.M. Nijssen, M.H. Boelens, Eds., 1994). The compound 3-hydroxy-5-methyl-2-hexanone has been found in water buffalo milk and its odour (GC sniffing) was described as melted cheese like (L. Moio, E. Semon, J.L. Le Quere, *Ital*. *J*. *Food Sci*. **1994,** *4*, 441).

Surprisingly it has been found that the two hydroxyketones of the invention have a green, pungent, roasted and cocoa like flavor. Mixtures thereof impart the pungent cocoa-like note of isovaleraldehyde to chocolate, cocoa and other products with roasted brown flavor. Therefore, the hydroxyketones of the present invention are useful for enhancing or modifying the flavor profile of roasted brown flavors, like cocoa, chocolate, coffee, caramel, toffee, roasted and regular nuts like hazelnuts, almonds, walnuts, chestnuts, macadamia, coconut, roasted butter, condensed milk, malt and the like. The compounds of the invention are especially useful to enhance the powdery and cocoa-like notes of cocoa and other roasted brown flavor notes.

Flavoring compositions with the roasted brown flavor profile enhanced or modified with the hydroxyketones of the invention are useful to impart the also freshly processed brown flavor qualities to food and beverage dairy products, e.g. UHT milk, condensed milk, yoghurt, cream desserts, cheese, bakery products, such as bread, biscuits, cookies, cakes, crackers, cereals, confectionery products like chocolate assortments, caramels, toffees, butterscotch.

Further, it has been found that the hydroxyketones of the present invention also act as masking agents in soybean derived products at the same time enhancing the flavor quality of these products. Soybean products like soybean milk, soybean yoghurt, tofu, etc. have a cereal and nut-like character, i.e. accompanied by a typical green vegetable note. Many consumers consider the green vegetable note as a drawback. Therefore, a masking agent for this typical green vegetable note in soybean products is of great value for the industry. The undesirable green, vegetable and beany off-flavor of soy protein has been reduced by treatment with porcine liver aldehyde oxidase (P. Maheshwari, P.A. Murphy, Z.L. Nikolov, *J. Agric. Food Chem.* ***1997*,** *45,* 2488), by treatment with liquid and supercritical carbon dioxide (P. Maheshwari, E.T. Ooi, Z.L. Nikolov, *J. Am*. *Oil Chem*. *Soc*. ***1995,*** *72,* 1107) and by fermentation with Bacillus subtilis and Bacillus natto (S.H. Choi, Y.A. Ji, *Korean J. Food Sci*. *Technol.* ***1989,*** *21*, 229).

Surprisingly, by the addition of the hydroxyketones of the present invention, not only the undesirable green, vegetable and beany notes of soybean products are remarkably reduced but at the same time the cereal nutty characteristics are enhanced. This results in a product with an overall more accepted flavor profile that is no longer associated with the negative green vegetable flavor of soybean derived products.

The compounds of the invention are also useful in food and beverage with other flavor characteristics, such as fruit products, diary products, vegetable products and meat products, spices, herbs, pharmaceutical products and oral hygene products.

The hydroxyketones of the invention can be given as such to the product or preferably as flavor composition comprising usual additives. When the hydroxyketones of the present invention are used in flavoring compositions to enhance modify or mask existing flavors or provide a characteristic impression, they may be incorporated into the flavoring compositions alone or in combination with additional flavor ingredients used for food products. Additional flavor ingredients which create roasted brown flavorings to be added to the flavor composition are esters, aldehydes, ketones, alcohols, lactones, heterocycles such as furans, pyridines, pyrazines and sulfur compounds like thiols, sulfides, disulfides and the like. These components can be combined in proportions normally used in the art for the preparation of flavoring.

When the hydroxyketones of the present invention are used in flavoring compositions having roasted brown flavor profile, especially in cocoa and chocolate flavoring compositions, to provide or modify the characteristic impression of isovaleraldehyde, they may be combined with isovaleraldehyde. Isovaleraldehyde usually present in chocolate, cocoa and other flavorings can be partially or completely substituted by the hydroxyketones of the invention. The combination of the compounds of the present invention with isovaleraldehyde is preferred for cocoa and chocolate flavoring compositions because of their milk chocolate like flavor.

The amount of hydroxyketones of the invention used depends on the precise organoleptic character desired in the finished product. In the case of flavoring compositions the amount of hydroxyketones will vary according to the food or beverage in which the flavor has to be enhanced or modified. The amount of hydroxyketones of the invention used also depends on the object, whether the roasted brown flavor profile of a product has to be enhanced or modified or full rounded roasted brown flavor has to be imparted to an unflavored material. In the later case more of the hydroxyketones of the invention are required. The ready to consume product with a roasted brown flavor preferably contains at least about 1 ppm of hydroxyketones of the invention based on the total weight of the product. It is generally desirable not to use more than about 20 ppm in the ready to consume product. The desirable range of flavoring composition in the ready to consume product corresponds to about 3 to about 6 ppm of the hydroxyketones of the invention. The amount of the flavoring composition added to the product should not only impart the desired flavor to the product but it should also give a balanced impression. Accordingly the flavoring composition of the invention preferably contains about 0,5 to about 1 % of the hydroxyketones of formula I and II based on the total weight of the flavoring composition.

For soybean products it is preferred to add at least about 0,1 ppm of the hydroxyketones of the invention, based on the total weight of the product. As upper limit 20 ppm is preferred. Flavor compositions for masking the undesired soybean flavor contain preferably about 0,5 to about 1 % of the hydroxyketones based on the total weight of the flavoring composition.

It may be desirable in the flavoring compositions of the invention to use carriers such as gum arabic, maltodextrin etc. or solvents such as ethanol, propyleneglycol, water, triacetin, etc. When the carrier is an emulsion, the flavoring composition may also contain emulsifiers such as mono- and diglycerides of fatty acids and the like. By using carriers or solvents the desired physical form of the flavoring composition can be obtained. The flavoring compositions of the invention can be used in spray-dried, liquid, encapsulated, emulsified or other forms.

The flavoring compositions of this invention can be added to food and beverage by conventional methods known to the skilled person. For example a chocolate praline may be prepared by incorporating the flavoring composition with fat, sugar, milk, cocoa powder and stabilizers and admixing these ingredients in a conventional freezer. A powdered chocolate or cocoa mix may be prepared by admixing dried milk solids, sugar and the flavoring composition in a dry blender until uniformity is obtained. In case of such dry mixtures the hydroxyketones or flavoring compositions can be added to one or more of the solid ingredients or to any portion thereof. In this case the flavoring composition may be spray-dried, encapsulated or the like.

A further object of the present invention is to provide a method for preparing the hydroxyketones of the invention in an economical way.

In the literature different methods for producing α-hydroxyketones are described. The JP-A2-04120043 discloses the synthesis of α-hydroxyketones by oxidizing olefins with H₂O₂ under acidic conditions. In the EP-A1-0 482 834 the preparation of α-hydroxyketones by oxidizing olefins with peracetic acids in the presence of a Ru catalyst is described. JP-A2-03167150 describes the synthesis of α-hydroxyketones by oxidation of alkynes with oxygen in the presence of silanes and a Co catalyst. In JP-86-150522 hydrolysis of α-halo arylketones in the presence of alkalihydroxides to prepare α-hydroxarylketones is described.

The present invention starts from inexpensive 5-methyl-2-hexanone which in a first step is reacted with sulfuryl chloride to give 3-chloro-5-methyl-2-hexanone with high selectivity.

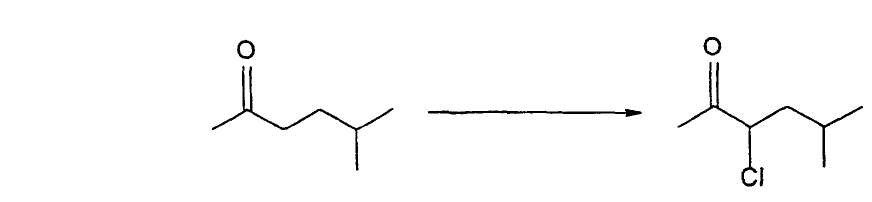

The 3-chloro-5-methyl-hexanone is treated in a second step with potassium acetate and alkali iodide.

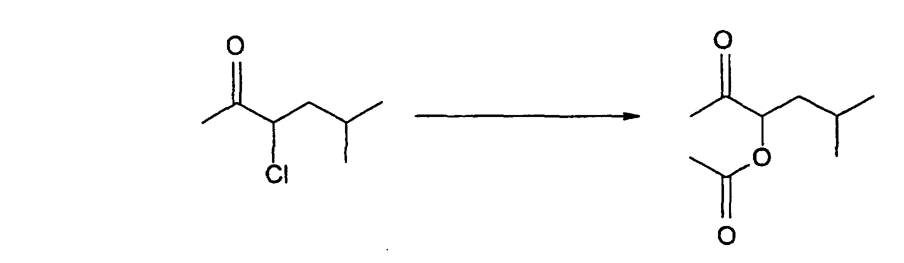

The 3-acetoxy-ketone obtained in the second step is hydrolized under alkaline conditions to give the desired 3-hydroxy-5-methyl-2-hexanone or a mixture of 3-hydroxy-5-methyl-2-hexanone and 2-hydroxy-5-methyl-3-hexanone.

The first step of the above synthesis is performed in presence or absence of an inert solvent such as hexane, cyclohexane, benzene, toluene and the like. Hexane is preferred. The reaction is carried out between room temperature and reflux temperature. The latter being preferred. The molar ratio of 5-methyl-2-hexanone to sulfuryl chloride may vary from 1:1 to 1:1,5 preferably 1:1,2. Molar ratios below 1,2 result in less chloroketone containing minor amounts of 3,3-dichloroketone. Molar ratios higher than 1,2 result in higher yields of chloroketone, however, formation of 3,3-dichloroketone increases. The product may be purified by distillation or directly used in the following step.

In the second step the 3-chloro-5-methyl-hexanone is treated with potassium acetate and an alkali iodide in an inert solvent, such as tetrahydrofuran, acetonitril, methylvinylketone, diethylketone, acetone and the like. The reaction is preferably run in acetone at the reflux temperature. Per mol potassium acetate 1:10 mol%, preferably 4 mol% of alkali iodide is used. Sodium iodide is preferred. The reaction mixture may be hydrolized with water or preferably is filtered to remove insoluble salts and washed with water and a sodium thiosulfate solution to remove traces of iodine. The crude product is purified by distillation under reduced pressure.

The 3-acetoxy-5-methyl-2-hexanone obtained in the second step is hydrolized in the third step with aqueous alkaline solution to give the desired mixtures of 3-hydroxy-5-methyl-2-hexanone and 2-hydroxy-5-methyl-3-hexanone. The hydrolysis is carried out between 0°C and reflux temperature depending on the desired ratio of isomeric hydroxyketones of formula I and II. If the reaction is run at elevated temperature and during a long reaction time using strong alkaline solution with a molar ratio of about 10 mol of a base per mol acetoxyketone an isomeric mixture containing approximately equal amounts of 3-hydroxy-5-methyl-2-hexanone and 2-hydroxy-5-methyl-3-hexanone is obtained. Using weak alkaline solution and long reaction time with a molar ratio of the base to acetoxyketone of 1.1 to 1 results in a mixture of higher amount of 3-hydroxy-5-methyl-2-hexanone.

The following examples show special embodiments of the invention without limiting it.

### Example 1

### 3(2)-Hydroxy-5-methyl-hexan-2(3)-one

To a refluxing solution (65°C) of 750 g (6.58 mol) 5-methyl-2-hexanone in 1.13 l *n*-hexane 1.06 kg (7.89 mol) sulfuryl chloride is added over a period of 2 h. After complete addition the reaction mixture is allowed to cool to room temperature, is washed 4 times with 150 ml H₂O, dried (MgSO₄) and concentrated *in vacuo*. The residue is distilled over a 5 cm *Vigreux*-column (80 mbar, 67-76°C) to give 884 g of 3-chloro-5-methyl-2-hexanone in form of a yellowish liquid (purity: 88%, impurity: 11% 3,3-dichloro-5-methyl-2-hexanone).

The chloroketone obtained is dissolved in 2 1 acetone and 588 g (6 mol) potassium acetate and 37.5 g (0.25 mol) sodium iodide is added. The mixture is heated at reflux under a N₂-atmosphere until complete conversion is observed (24 h), then it is cooled to room temperature and filtered. The residue is washed with 250 ml acetone and the combined filtrates are concentrated in vacuo. The residue is taken up in 1 1 MTBE (Methyl-tert-butyl ether) and filtered. The filtrate is washed with 600 ml of sodium thiosulfate solution (10%) and 3 times with 200 ml H₂O, dried (MgSO₄) and concentrated *in vacuo*. The crude product is distilled using a 20 cm *Widmer*-column (18 mbar, 92-96°C) to give 750 g (64% yield over two steps) of 3-acetoxy-5-methyl-2-hexanone (97% purity).

To a 0°C cold solution of 580 g (3.37 mol) 3-acetoxy-5-methyl-2-hexanone in 680 ml methanol, a solution of 513 g (3.7 mol) potassium carbonate in 680 ml H₂O is added under vigorous stirring over a period of 1.5 h. The reaction mixture is allowed to warm overnight to room temperature. The solid potassium carbonate is removed by filtration and washed with 100 ml of MTBE. The filtrate is concentrated *in vacuo* and the residue is taken up in 300 ml MTBE. The organic layer is separated, washed 3 times with 150 ml brine and 1 time with 200 ml H₂O, dried (MgSO₄) and concentrated *in vacuo* to give 428 g (97%) crude product. Distillation using a 18 cm *Widmer*-column (0.06 torr, 40°C) gives 330 g (75%) 3(2)-hydroxy-5-methyl-hexan-2(3)-one. Isomeric ratio: 78.2:21.8.

### 3-Hydroxy-5-methyl-hexan-2-one

NMR (400 Mhz, CDCl₃): 0.92 (*d*, *J* = 6.8 Hz, CH₃), 0.97 (*d*, *J* = 6.8 Hz, CH₃), 1.18-1.22 (*m*, CH₂); 1.61 (*s*, CH₃); 2.05 (*m*, CH(CH₃)₂); 3.50 (*d*, *J* = 4.8 Hz, OH); 3.87 (*m*, CHOH). MS: 130 (0.1, M⁺), 87 (37), 74 (17), 69 (73), 57 (13), 45 (52), 43 (100), 41 (31), 29 (5).

### 2-Hydroxy-5-methyl-hexan-3-one

NMR (400 Mhz, CDCl₃): 0.80 (*d*, *J* = 6.8 Hz, CH₃), 0.82 (*d*, *J* = 6.8 Hz, CH₃), 1.11 (*d*, *J* = 7.2 Hz, CH₃); 1.75 (*dd*, *J =* 6.8, 16.8, 1H, CH₂); 1.92 (*dd*, *J* = 6.8, 16.8, 1H, CH₂); 2.15 (*m*, CH(CH₃)₂); 3.65 (*d*, *J* = 4.8 Hz, OH); 3.89 (*m*, CHOH). MS: 130 (0.1, M⁺), 85 (90), 74 (13), 69 (22), 57 (100), 45 (79), 43 (39), 41 (37), 29 (14).

### Example 2

A flavoring composition having a typical chocolate flavor was prepared using the following ingredients:

| Ingredient | Parts by weight | |
|---|---|---|
| | A | B |
| Benzaldehyde | 0.02 | 0.02 |
| Cocoa extract | 85.88 | 85.38 |
| Ethyl phenyl acetate | 0.15 | 0.15 |
| Ethylvanillin | 5.00 | 5.00 |
| *iso*-Butyric acid | 0.05 | 0.05 |
| Phenyl acetic acid | 0.30 | 0.30 |
| Phenyl-ethyl alcohol | 0.20 | 0.20 |
| 2,3,5-Trimethylpyrazine | 0.20 | 0.20 |
| 2-Ethyl-3,5-dimethyl-pyrazine | 0.20 | 0.20 |
| Vanillin | 8.00 | 8.00 |
| 3(2)-Hydroxy-5-methyl-hexan-2(3)-one | - | 0.50 |
| Total | 100.00 | 100.00 |

The chocolate flavorings A and B have been added at 0.07% to commercial pasturized milk (3.5% fat content) sweetened with 6% sugar. The thus prepared chocolate flavored milk drinks A and B have been evaluated in a blind test by an expert panel of flavorists. The panel judged the chocolate milk drink B to have enhanced cocoa and powdery notes, to give a more pronounced nice dark chocolate impression and to have an overall improved taste profile.

### Example 3

A flavoring composition having a coconut flavor was prepared using the following ingredients:

| Ingredient | Parts by weight | |
|---|---|---|
| | A | B |
| Ethylvanillin | 0.1 | 0.1 |
| γ-Nonalactone | 1.0 | 1.0 |
| Propylene glycol | 60.9 | 60.4 |
| Water | 38.0 | 38.0 |
| 3(2) -Hydroxy-5-methyl-hexan-2 (3)-one | - | 0.5 |
| Total | 100.0 | 100.0 |

The coconut flavorings A and B have been added at 0.06% to commercial pasturized milk (3.5% fat content) sweetened with 6% sugar. The thus prepared coconut flavored milk drinks A and B have been evaluated in a blind test by an expert panel of flavorists. The panel judged the coconut milk drink B to have a more fresh coconut flesh (white part of the coconut) character and an enhanced coconut milk taste profile.

### Example 4

A flavoring composition having a roasted hazelnut flavor was prepared using the following ingredients:

| Ingredient | Parts by weight | |
|---|---|---|
| | A | B |
| 2 -Acetyl-pyrazine | 0.2 | 0.2 |
| Benzaldehyde | 0.2 | 0.2 |
| Diacetyl | 0.3 | 0.3 |
| Dimethyl-resorcinol | 0.2 | 0.2 |
| Methylcorylone | 0.6 | 0.6 |
| 5-Methyl-furfural | 0.4 | 0.4 |
| γ-Nonalactone | 0.1 | 0.1 |
| Propylene glycol | 95.9 | 94.9 |
| 2,3,5-Trimethylpyrazine | 0.1 | 0.1 |
| Vanillin | 2.0 | 2.0 |
| 3(2) -Hydroxy-5-methyl-hexan-2 (3)-one | - | 1.0 |
| Total | 100.0 | 100.0 |

The hazelnut flavorings A and B have been added at 0.05% to commercial pasturized milk (3.5% fat content) sweetened with 6% sugar. The thus prepared hazelnut flavored milk drinks A and B have been evaluated in a blind test by an expert panel of flavorists. The panel judged the hazelnut milk drink B to have a more rounded off roasted hazelnut flavor, a reduced harshness of pyrazine and resorcinol character and an enhanced freshly roasted and nutty character.

### Example 5

A commercially available UHT soybean drink (Vegi Line Sojadrink, Migros MGB, CH-8031 Zürich) was flavored with 0.5 ppm 3(2)-hydroxy-5-methyl-hexan-2(3)-one. The thus prepared soybean drink A and the unflavored soybean drink B have been evaluated in a blind test by an expert panel of flavorists. The panel judged the flavored soybean drink B to be much more accepted because the undesirable green/vegetable notes typically associated with soybeans are remarkably reduced, giving the drink an overall pleasant flavor profile with enhanced milky/creamy and cereal/nutty notes.

### Example 6

A flavoring composition having a banana flavor was prepared using the following ingredients:

| Ingredient | Parts by weight | |
|---|---|---|
| | A | B |
| Canaga oil | 0.005 | 0.005 |
| Clove leaf oil | 0.020 | 0.020 |
| Ethyl acetate | 0.125 | 0.125 |
| Furaneol (15% in PG) | 0.133 | 0.133 |
| Isoamyl acetate | 3.470 | 3.470 |
| Isoamyl alcohol | 0.125 | 0.125 |
| Isoamyl caproate | 0.200 | 0.200 |
| Isobutyl acetate | 0.600 | 0.600 |
| Isobutyl alcohol | 0.400 | 0.400 |
| Linalool | 0.0025 | 0.0025 |
| Banana Ess. 50x | 0.100 | 0.100 |
| Acetaldehyde | 0.025 | 0.025 |
| Propylene glycol | 94.817 | 94.717 |
| 3(2)-Hydroxy-5-methyl-hexan-2(3)-one | - | 0.100 |
| Total | 100.0 | 100.0 |

The banana flavorings A and B have been added at 0.1% to water sweetened with 5% sugar. The thus prepared banana flavorings A and B have been evaluated in a blind test by an expert panel of flavorists. The panel judged the banana flavoring B to be fuller in body and to have a more true-fruit-like and more ripe character.

## Claims

1. 2-Hydroxy-5-methyl-hexan-3-one.

2. A mixture of 3-hydroxy-5-methyl-hexan-2-one and 2-hydroxy-5-methyl-hexan-3-one.

3. A flavoring and/or masking composition comprising 3-hydroxy-5-methyl-hexan-2-one and 2-hydroxy-5-methyl-hexan-3-one.

4. The flavoring and/or masking composition according to claim 3 wherein the ratio of 3-hydroxy-5-methyl-hexan-2-one to 2-hydroxy-5-methyl-hexan-3-one varies from 1:1 to 99:1.

5. The flavoring and/or masking composition according to claim 4 comprising additional organoleptic compounds, carriers and/or vehicles.

6. Food or beverage comprising 3-hydroxy-5-methyl-hexan-2-one and 2-hydroxy-5-methyl-hexan-3-one as flavoring and/or masking agent.

7. Food or beverage according to claim 6 comprising 3-hydroxy-5-methyl-hexan-2-one and 2-hydroxy-5-methyl-hexan-3-one in an amount sufficient to impart a green pungent cocoa-like flavor quality to the food or beverage.

8. Food or beverage according to claim 6 or 7 comprising a soybean-based product and 3-hydroxy-5-methyl-hexan-2-one and 2-hydroxy-5-methyl-hexan-3-one.

9. Process for the preparation of a mixture of 3-hydroxy-5-methyl-hexan-2-one and 2-hydroxy-5-methyl-hexan-3-one by reacting 5-methyl-2-hexanone with sulfuryl chloride to 3-chloro-5-methyl-2-hexanone, reacting the latter with potassium acetate and alkali iodide in an inert solvent to 3-acetoxy-5-methyl-2-hexanone and hydrolyzing the acetoxy compound under strong alkaline conditions.

## Patentansprüche

1. 2-Hydroxy-5-methyl-hexan-3-on,

2. Ein Gemisch aus 3-Hydroxy-5-methyl-hexan-2-on und 2-Hydroxy-5-methyl-hexan-3-on,

3. Geschmackstoff- und/oder maskierende Zusammensetzung, die 3-Hydroxy-5-methyl-hexan-2-on und 2-Hydroxy-5-methyl-hexan-3-on umfasst,

4. Geschmackstoff- und/oder maskierende Zusammensetzung gemäß Anspruch 3, wobei das Verhältnis von 3-Hydroxy-5-methyl-hexan-2-on zu 2-Hydroxy-5-methyl-hexan-3-on von 1:1 bis 99:1 schwankt,

5. Geschmackstoff- und/oder maskierende Zusammensetzung nach Anspruch 4, die weitere organoleptische Verbindungen, Träger und/oder Vehikel umfasst,

6. Nahrungsmittel oder Getränk, das 3-Hydroxy-5-methyl-hexan-2-on und 2-Hydroxy-5-methyl-hexan-3-on als Geschmackstoff- und/oder maskierendes Mittel umfasst,

7. Nahrungsmittel oder Getränk nach Anspruch 6, das 3-Hydroxy-5-methyl-hexan-2-on und 2-Hydroxy-5-methylhexan-3-on in einer ausreichenden Menge umfasst, um dem Nahrungsmittel oder Getränkt eine grüne scharfe kakaoartige Geschmackstoffqualität zu verleihen,

8. Nahrungsmittel oder Getränk nach Anspruch 6 oder 7, die ein Produkt auf Sojabohnenbasis und 3-Hydroxy-5-methyl-hexan-2-on und 2-Hydroxy-5-methyl-hexan-3-on umfasst,

9. Verfahren zur Herstellung eines Gemisches von 3-Hydroxy-5-methyl-hexan-2-on und 2-Hydroxy-5-methylhexan-3-on durch Umsetzen von 5-Methyl-2-hexanon mit Sulfurylchlorid zu 3-Chlor-5-methyl-2-hexanon, Umsetzen der letzteren Verbindung mit Kaliumacetat und Alkaliiodid in einem inerten Lösemittel zu 3-Acetoxy-5-methyl-2-hexanon und Hydrolisieren der Acetoxyverbindung unter starken alkalischen Bedingungen.

## Revendications

1. 2-Hydroxy-5-méthyl-hexan-3-one.

2. Mélange de 3-hydroxy-5-méthyl-hexan-2-one et de 2-hydroxy-5-méthyl-hexan-3-one.

3. Composition aromatisante et/ou masquante comprenant de la 3-hydroxy-5-méthyl-hexan-2-one et de la 2-hydroxy-5-méthyl-hexan-3-one.

4. Composition aromatisante et/ou masquante selon la revendication 3, dans laquelle le rapport de la 3-hydroxy-5-méthyl-hexan-2-one à la 2-hydroxy-5-méthyl-hexan-3-one varie de 1:1 à 99:1.

5. Composition aromatisante et/ou masquante selon la revendication 4, comprenant des composés organoleptiques additionnels, des supports et/ou des véhicules.

6. Aliment ou boisson comprenant de la 3-hydroxy-5-méthyl-hexan-2-one et de la 2-hydroxy-5-méthyl-hexan-3-one en tant qu'agent aromatisant et/ou masquant.

7. Aliment ou boisson selon la revendication 6, comprenant de la 3-hydroxy-5-méthyl-hexan-2-one et de la 2-hydroxy-5-méthyl-hexan-3-one en quantité suffisante pour conférer une qualité d'arôme vert relevé de type cacao à l'aliment ou à la boisson.

8. Aliment ou boisson selon la revendication 6 ou 7, comprenant un produit à base de soja ainsi que de la 3-hydroxy-5-méthyl-hexan-2-one et de la 2-hydroxy-5-méthyl-hexan-3-one.

9. Procédé de préparation d'un mélange de 3-hydroxy-3-méthyl-hexan-2-one et de 2-hydroxy-5-méthyl-hexan-3-one par réaction de la 5-méthyl-2-hexanone avec du chlorure de sulfuryle pour donner la 3-chloro-5-méthyl-2-hexanone, réaction de cette dernière avec de l'acétate de potassium et un iodure de métal alcalin dans un solvant inerte pour donner la 3-acétoxy-5-méthyl-2-hexanone et hydrolyse du composé acétoxy dans des conditions fortement alcalines.
